# DEMANDE DE BREVET EUROPEEN

(11) **EP 0 719 562 A1**
(43) Date de publication de la demande: **03.07.1996**
(21) Numéro de dépôt: 95402911.2
(22) Date de dépôt: 21.12.1995
(51) Int. Cl.: A61L 27/00

(54) **Endoprothèse avec revêtement en calcite et procédé d'obtention**

(30) Priorité: 30.12.1994 FR 9415955
(71) Demandeur: DIAMI, F-31370 Rieumes (FR)
(72) Inventeur: Perisse, Pierre, F-31370 Rieumes (FR); Deloison, Victor, F-92400 Courbevoie (FR)
(74) Mandataire: Phélip, Bruno

(57) **Abrégé**

L'invention concerne une endoprothèse destinée à être fixée sans ciment dans un corps vivant, humain ou animal, et présentant un revêtement réalisé en carbonate de calcium, de type calcite.

Elle concerne également un procédé pour l'obtention d'un revêtement en calcite sur une endoprothèse.

## Description

La présente invention concerne le domaine des endoprothèses, en particulier les implants dentaires, les implants orthopédiques (hanche, genou...) et traumatologiques (vis, plaque).

Elle a notamment pour objet une endoprothèse présentant un nouveau type de revêtement et un procédé pour l'obtention d'un tel revêtement.

Différents moyens sont déjà connus pour fixer une endoprothèse dans un corps vivant, humain ou animal.

Ainsi, les endoprothèses ont classiquement été fixées avec un ciment. Plusieurs années après la mise en place de l'endoprothèse, celle-ci peut se desceller. Il faut alors procéder à une nouvelle opération car le patient souffre. On constate que le retrait du ciment est parfois difficile.

Depuis une trentaine d'années, des recherches sont menées pour mettre au point des endoprothèses pouvant être fixées sans utilisation de ciment.

Ces recherches ont conduit à réaliser des revêtements sur l'endoprothèse elle-même, constituant un substrat.

Il a déjà été mis au point un revêtement d'hydroxyapatite, qui présente des inconvénients.

Tout d'abord ce revêtement est classiquement réalisé au moyen d'une torche à plasma. Celle-ci projette une poudre sous forme de plasma, à des températures très élevées (10.000 - 12.000°C). Le plasma est projeté sur le substrat pour constituer le revêtement.

Cette technique est relativement complexe et onéreuse.

On constate également que le comportement dans le temps d'une endoprothèse, avec un revêtement en hydroxyapatite, n'est pas toujours satisfaisant.

Quelques mois après la mise en place de la prothèse, il peut, dans certains cas, se former un tissu fibreux entre l'os et la prothèse. Il a été avancé que c'est l'absorption de l'hydroxyapatite par l'os qui pourrait conduire à la formation de ce tissu fibreux. Celui-ci affaiblit la fixation de l'endoprothèse dans l'os.

L'invention a donc pour objet de pallier les inconvénients des techniques connues, en proposant un nouveau revêtement pour endoprothèse présentant un pouvoir ostéoconducteur et résistant à la dissolution, une fois implanté dans l'os. Le procédé d'obtention de ce revêtement est, en outre, simple et peu coûteux.

L'invention concerne une endoprothèse destinée à être fixée sans ciment dans un corps vivant, humain ou animal, et présentant un revêtement réalisé en carbonate de calcium, du type calcite.

En effet, le carbonate de calcium présente différentes formes cristallographiques: calcite, aragonite et vatérite, dont les comportements sont différents.

Le carbonate de calcium a déjà été utilisé dans d'autres domaines, par exemple comme matériau de comblement, pour constituer un substitut osseux.

On peut notamment citer les implants à base de corail, qui est du carbonate de calcium de type aragonite. Ces implants sont remplacés par de l'os naturel au fur et à mesure de leur dégradation dans l'organisme.

Jusqu'à présent, il n'a jamais été envisagé de réaliser des revêtements d'endoprothèse en calcite.

Dans le cadre de l'invention, on a pu constater que les revêtements de calcite permettent la repousse osseuse. Les tests de résistance à la traction ont également montré que la qualité de la fixation de la calcite sur le substrat constitué par l'endoprothèse, est environ cinq fois supérieure à celle de l'hydroxyapatite.

Les essais réalisés ont mis en évidence des caractéristiques préférentielles de l'invention, qui peuvent être prises isolément ou en combinaison:
- l'épaisseur du revêtement est comprise entre 50 et 200 µm, et notamment entre 100 et 150 µm
- le revêtement est en calcite pure.

L'endoprothèse constitue un substrat réalisé dans des matériaux classiques pour ce type d'application. On peut notamment citer l'acier inoxydable, les alliages au chrome-cobalt, les alliages de titane, les matériaux biodégradables.

L'invention concerne également un procédé d'obtention d'un revêtement en carbonate de calcium, du type calcite, sur une endoprothèse.

Selon le procédé, on constitue un milieu aqueux de concentration déterminée en carbonate de calcium de type calcite, puis on immerge l'endoprothèse dans ledit milieu pendant une durée déterminée, du carbonate de calcium du type calcite se dépose alors sur le substrat pour former ledit revêtement, la durée d'immersion étant fonction de la concentration en calcite dudit milieu aqueux et de l'épaisseur souhaitée pour le revêtement.

Ce procédé présente l'avantage d'être très simple à mettre en oeuvre puisqu'il consiste essentiellement à mettre un substrat en contact avec un milieu aqueux contenant du carbonate de calcium de type calcite, en proportion déterminée.

Il permet également d'éviter tout changement physico-chimique du matériau déposé, lors de la constitution du revêtement. Ceci est avantageux par rapport aux revêtements d'hydroxyapatite qui sont réalisés à partir de poudres transformées en plasma, sous l'effet de la température.

Des caractéristiques préférentielles de l'invention ont été mises en évidence, elles peuvent être prises isolément ou en combinaison:
- dans un milieu aqueux saturé en carbonate de calcium de type calcite, la durée d'immersion de l'endoprothèse est comprise entre 40 et 48 heures, le revêtement obtenu présentant une épaisseur comprise entre 150 et 200 µm,
- le milieu aqueux est constitué à partir d'eau pure, c'est-à-dire exempte d'éléments étrangers toxiques et dont les constituants minéraux autres que le calcium sont, de préférence, présents sous forme de traces, ou encore à des teneurs inférieures à 0,5 g/l,
- le milieu aqueux est constitué à partir d'eau préalablement soumise à une filtration ou à une osmose inverse,
- le milieu aqueux est maintenu à une température d'environ 37°C,
- l'immersion est effectuée sous agitation, pour obtenir un revêtement sensiblement uniforme,
- le milieu aqueux est périodiquement rechargé en carbonate de calcium du type calcite.

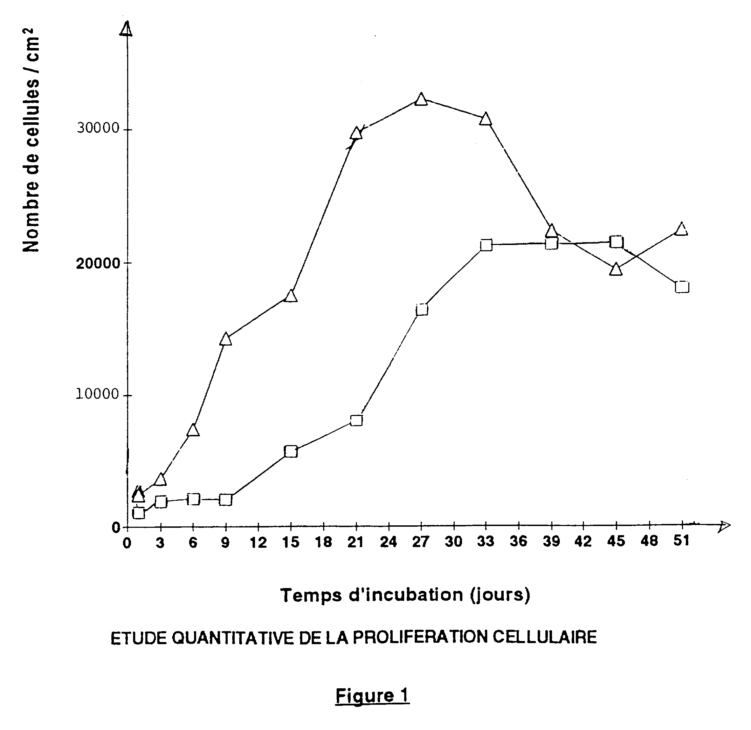

Les caractéristiques de l'endoprothèse selon l'invention et du procédé d'obtention d'un revêtement de carbonate de calcium du type calcite, ainsi que leurs avantages apparaitront plus clairement des résultats d'essais qui sont maintenant rapportés, notamment au regard de la figure 1 qui est un diagramme représentant la croissance des ostéoblastes humains en fonction du temps, pour un revêtement de calcite (représenté par - -) et pour un revêtement d'hydroxyapatite (représenté par - -).

En premier lieu, la nature du revêtement obtenu par le procédé selon l'invention sur un substrat peut être caractérisée, notamment par diffraction des rayons X ou spectroscopie infra-rouge.

La première de ces analyses s'effectue directement sur le revêtement par diffraction des rayons X.

Un appareil approprié, notamment un générateur SIGMA 2060, permet d'obtenir un diagramme présentant les raies de la forme calcite du carbonate de calcium.

Pour réaliser l'analyse par spectroscopie infra-rouge, il convient tout d'abord de prélever le revêtement présent sur le substrat. Celui est ensuite préparé dans des conditions appropriées et analysé dans un appareil à Transformée de Fourier, par exemple du type Elmer 1600.

La spectroscopie infra-rouge révèle la présence d'ions carbonate dans la structure calcite avec des bandes caractéristiques à 712, 874 et 1421 cm⁻¹.

Ces deux types d'analyse montrent que le revêtement obtenu par le procédé selon l'invention est de préférence constitué d'une seule phase de structure calcite, bien cristallisée.

Il a été précédemment indiqué que le milieu aqueux, utilisé pour la mise en oeuvre du procédé selon l'invention, était de préférence constitué à partir d'eau exempte de produits toxiques. En effet, l'endoprothèse selon l'invention est destinée à être placée dans le corps humain. Il est donc nécessaire que le revêtement réalisé ne contienne aucun élément toxique qui pourrait notamment se fixer au niveau osseux.

A cet égard et à titre illustratif, on peut se référer à la norme NF S 94-065 qui concerne le dosage du Mercure, du Plomb, de l'Arsenic et du Cadmium sur des revêtements à base de phosphate de calcium pour prothèses orthopédiques. Ces quatre éléments sont toxiques et leur teneur doit donc être contrôlée.

De préférence, les constituants minéraux autres que le calcium, par exemple le magnésium, sont présents dans l'eau utilisée sous forme de traces, c'est-à-dire à des teneurs inférieures à 0,5 g/l.

Si des constituants minéraux autres que le calcium, sont présents dans l'eau utilisée ou dans la calcite qui y est dissoute, ces constituants seront également au moins partiellement présents dans le revêtement obtenu. Leur présence pourra d'ailleurs être mise en évidence par une analyse par diffraction des rayons X ou par spectroscopie infra-rouge.

Pour différentes raisons, et notamment pour assurer la biocompatibilité de l'endoprothèse selon l'invention, un revêtement de calcite sensiblement pure peut être préféré.

Comme indiqué précédemment, l'eau utilisée sera exempte de produits toxiques pour éviter leur présence dans le revêtement.

De façon générale, la pureté du revêtement obtenu sera fonction de la pureté de l'eau utilisée et de celle du carbonate de calcium que l'on y dissout pour constituer le milieu aqueux dans lequel la prothèse est immergée. En effet, des impuretés peuvent précipiter, lors de la cristallisation, avec le calcium ou le carbonate (par exemple le magnésium).

Ainsi, l'eau utilisée peut être purifiée par osmose inverse ou par filtration. Cependant, le taux de pureté ainsi obtenu n'est pas absolument nécessaire pour obtenir un revêtement compatible avec des exigences cliniques.

Les essais réalisés ont eu tout d'abord pour objet de montrer qu'un revêtement en carbonate de calcium, du type calcite, a effectivement un pouvoir ostéoconducteur.

Pour cela, des ostéoblastes humains sont mis en contact direct avec des échantillons constitués par un substrat en alliage de titane, dénommé TA6V, présentant un revêtement de carbonate de calcium, du type calcite.

La présence de cellules osseuses, après quelques jours d'incubation, est tout d'abord montrée grâce à l'observation des échantillons par microscopie électronique à balayage.

La croissance cellulaire a également été mise en évidence par une étude quantitative de l'attachement et de la prolifération des ostéoblastes suivant la norme NF S 91-142.

Un exemple des résultats obtenus est illustré à la figure 1.

Des échantillons de dimensions données présentant un revêtement de calcite ont été mis en contact avec des ostéoblastes humains pendant une durée de 51 jours, la densité cellulaire initiale étant de 5 200 cellules/cm². On observe un temps d'adaptation puis une prolifération continue jusqu'au 33ème jour d'incubation, où la densité cellulaire reste stable jusqu'au 51ème jour à environ 20 000 cellules/cm².

Des essais ont également été effectués dans les mêmes conditions expérimentales avec des échantillons de même nature et de mêmes dimensions, mais présentant un revêtement d'hydroxyapatite. On observe une prolifération continue jusqu'au 21ème jour d'incubation environ, ensuite un plateau pour une densité cellulaire d'environ 30 000 cellules/cm², puis une perte cellulaire qui semble se stabiliser à 20 000 cellules/cm² après 45 jours d'incubation.

On peut ainsi constater que sur un revêtement de calcite, la croissance de cellules osseuses est continue et se réalise selon une courbe de prolifération normale. Au contraire, avec un revêtement d'hydroxyapatite, on observe une chute de la densité cellulaire.

Ainsi, le comportement des ostéoblastes est plus homogène, sur un revêtement du type calcite que sur un revêtement d'hydroxyapatite.

On a également étudié la différenciation cellulaire de l'ostéoblaste humain sur des substrats comportant un revêtement de carbonate de calcium, de type calcite. Le paramètre retenu est la phosphatase alcaline cellulaire qui est un enzyme spécifique de l'ostéoblaste.

Les substrats à revêtement de calcite ont été comparés à des substrats de même nature, mais comportant un revêtement d'hydroxyapatite, obtenu avec une technique du type torche à plasma.

On a pu alors mettre en évidence une stimulation de l'activité de la phosphatase alcaline cellulaire sur un revêtement de calcite. Ceci permet de penser qu'un revêtement de calcite conduit à une meilleure différenciation cellulaire de l'ostéoblaste humain qu'un revêtement en hydroxyapatite.

Ceci constitue un avantage majeur du revêtement en calcite par rapport aux revêtements connus en hydroxyapatite.

D'autres essais ont consisté à comparer la biodégradation de substrats comportant d'une part, un revêtement d'hydroxyapatite et d'autre part, un revêtement de calcite.

Ils ont montré qu'à pH neutre, la calcite présente une dissolution moins importante que l'hydroxyapatite. On a également procédé à des essais de traction sur des revêtements en calcite en les comparant à des revêtements d'hydroxyapatite. Les résultats obtenus montrent que la résistance à la traction est de 5 à 6 fois supérieure pour un revêtement de calcite que pour un revêtement d'hydroxyapatite.

Ainsi, sur la base des résultats obtenus dans les essais de traction et de biodégradation, on peut penser que les risques de formation de tissus fibreux, entre l'os et l'endoprothèse présentant un revêtement de calcite, sont moindres que lorsque un revêtement d'hydroxyapatite est utilisé.

Enfin, des essais ont porté sur l'épaisseur du revêtement de calcite déposé sur l'endoprothèse.

Ils ont mis en évidence que l'épaisseur minimale du revêtement est, de préférence, de 50 µm et que l'épaisseur maximale est, de préférence, de 200 µm.

La gamme d'épaisseur préférée est comprise entre 100 et 150 µm. C'est dans cette gamme que le revêtement de calcite semble le mieux se comporter, notamment en ce qui concerne la biodégradation.

Le procédé d'obtention selon l'invention va maintenant être décrit plus en détail.

On constitue tout d'abord un milieu aqueux contenant du carbonate de calcium du type calcite, en proportion déterminée.

Comme on le verra plus loin, la concentration en carbonate de calcium est choisie en fonction de la durée du procédé et de l'épaisseur du revêtement souhaitées. On utilise, de préférence, un milieu aqueux saturé en calcite.

L'eau utilisée est, de préférence, de l'eau pure, c'est-à-dire exempte de produits toxiques. Ceci est notamment justifié pour assurer la biocompatibilité de l'endoprothèse selon l'invention.

De préférence également, les composés minéraux présents dans l'eau utilisée le sont sous forme de traces, c'est-à-dire à des teneurs inférieures à 0,5 g/l.

Comme indiqué précédemment, la présence de composants minéraux autres que le calcium peut être mise en évidence au moyen d'analyse par diffraction de rayons X ou par spectroscopie infra-rouge.

L'endoprothèse à traiter est ensuite immergée dans le milieu aqueux de concentration en calcite déterminée. La calcite se dépose alors sur le substrat. La durée d'immersion dépend de la concentration en calcite et de l'épaisseur souhaitée pour le revêtement.

A titre d'exemple, on a pu mettre en évidence que, pour un milieu aqueux saturé en calcite et une durée d'immersion comprise entre 40 et 48 heures, l'épaisseur du revêtement est comprise entre 150 et 200 µm.

Comme indiqué précédemment, une analyse du revêtement par diffraction des rayons X ou par spectrographie infra-rouge montre que le revêtement obtenu est bien constitué par de la calcite. Le degré de pureté de la calcite dépend de la pureté de l'eau et de la calcite utilisées.

De préférence, l'immersion se réalise sous agitation, celle-ci étant obtenue par des moyens classiques. Ainsi, le dépôt de calcite peut atteindre toutes les zones du substrat et le revêtement obtenu est sensiblement uniforme.

De manière avantageuse, le milieu aqueux est périodiquement rechargé en carbonate de calcium, de structure calcite. Ceci contribue à uniformiser dans le temps, la quantité de calcite qui se dépose sur le substrat.

La température du milieu aqueux est également contrôlée lors de la mise en oeuvre du procédé. En effet, la repousse osseuse est favorisée lorsque les cristaux sont de taille relativement faible. Or, la taille des germes à l'origine des cristaux dépend de la température du milieu.

On a constaté qu'une taille appropriée de cristaux était obtenue lorsque la température du milieu aqueux était voisine de 37°C.

Ceci met en évidence un autre avantage de l'invention puisque la mise en oeuvre du procédé évite toute modification physico-chimique, contrairement au procédé par torche à plasma, utilisé pour réaliser les revêtements d'hydroxyapatite.

## Revendications

1. Endoprothèse destinée à être fixée sans ciment dans un corps vivant, humain ou animal, caractérisée en ce qu'elle présente un revêtement réalisé en carbonate de calcium, du type calcite.

2. Endoprothèse selon la revendication 1, caractérisée en ce que ledit revêtement est compris entre 50 et 200 µm, notamment entre 100 et 150 µm.

3. Endoprothèse selon l'une des revendications 1 ou 2, caractérisée en ce que le revêtement est réalisé en calcite pure.

4. Endoprothèse selon l'une des revendications 1 à 3, caractérisée en ce que le substrat est réalisé en acier inoxydable, en alliage au chrome-cobalt, en alliage de titane ou encore en matériaux biodégradables.

5. Procédé d'obtention d'un revêtement en carbonate de calcium, du type calcite, sur une endoprothése, selon lequel:
- on constitue un milieu aqueux contenant une concentration déterminée de carbonate de calcium du type calcite,
- on immerge l'endoprothèse dans ledit milieu pendant une durée déterminée, du carbonate de calcium du type calcite se déposant alors sur le substrat pour former ledit revêtement, la durée d'immersion étant fonction de la concentration en calcite dudit milieu aqueux et de l'épaisseur souhaitée pour le revêtement.

6. Procédé selon la revendication 5, dans lequel le milieu aqueux est saturé en calcite.

7. Procédé selon l'une des revendications 5 ou 6, dans lequel, le milieu aqueux étant saturé en carbonate de calcium de type calcite, la durée d'immersion de l'endoprothèse est comprise entre 40 et 48 heures, le revêtement obtenu présentant une épaisseur comprise entre 150 et 200 µm.

8. Procédé selon l'une des revendications 5 à 7, le milieu aqueux étant constitué à partir d'eau pure, c'est-à-dire exempte de produits toxiques et, de préférence, dont les constituants minéraux autres que le calcium sont présents sous forme de traces.

9. Procédé selon l'une des revendications 5 à 8, le milieu aqueux étant constitué à partir d'eau préalablement soumise à une filtration ou à une osmose inverse.

10. Procédé selon l'une des revendications 5 à 9, le milieu aqueux étant maintenu à une température d'environ 37°C.

11. Procédé selon l'une des revendications 5 à 10, l'immersion étant effectuée sous agitation.

12. Procédé selon l'une des revendications 5 à 11, le milieu aqueux étant périodiquement rechargé en carbonate de calcium, du type calcite.
